# EUROPEAN PATENT APPLICATION

(11) **EP 2 113 504 A1**
(43) Date of publication of application: **04.11.2009**
(21) Application number: 08155371.1
(22) Date of filing: 29.04.2008
(51) Int. Cl.: C07D 457/02, C07D 457/04, C07D 457/06, A61K 31/48, A61P 25/00

(54) **Preparation of N-6 demethylated, 9,10-dihydrolysergic acid alkyl esters**

(71) Applicant: LEK Pharmaceuticals d.d., 1526 Ljubljana (SI)
(72) Inventor: The designation of the inventor has not yet been filed
(74) Representative: Demsar, Marija

(57) **Abstract**

A process for producing *N*-6 demethylated 9,10-dihydrolysergic acid alkyl ester is described, which comprises a step of *N*-6 demethylation of 9,10-dihydrolysergic acid alkyl esters with chloroformate and wherein the *N*-6 demethylation step is performed in the presence of an organic catalyst. Further, a process for converting a compound of formula I (DHLSAIkyI) into a compound of formula II (DHLSAIkyI-F) comprises the steps of: providing (and maintaining) a reaction medium free of water and/or HCI, and reacting DHLSAIkyI with chloroformate in said reaction medium free of water and/or HCI, to obtain the compound of formula II (DHLSAIkyI-F).

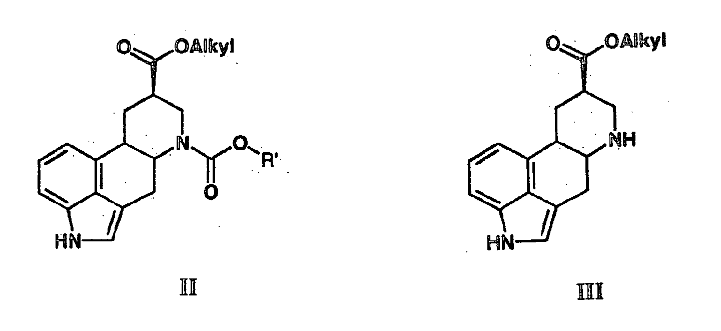

## Description

### Field of the Invention

The present invention relates to a process for producing N-6 demethylated 9,10-dihydrolysergic acid alkyl esters comprising a step of N-6 demethylation of 9,10-dihydrolysergic acid alkyl esters with chloroformates, which may form a step in the synthesis of ergot alkaloid drugs. The present invention further relates to pure ergot alkaloid drugs and intermediate products therefor.

### Background of the Invention

The N-6 demethylation of 9,10-dihydrolysergic acid alkyl esters (in the following sometimes abbreviated as DHLSAlkyl, for example its methylester (DHLSMe), may constitute a useful step in the chemical synthesis of various ergot alkaloid drugs which have a range of medical activities. A convenient synthesis for the N-6 demethylation was disclosed a few decades ago by A. M. Crider et al. in Journal of Pharmaceutical Sciences, Vol. 70 (12), pp. 1319-1321 (1981*)*.

The object of the present invention is to provide an improved process for producing N-6 demethylated 9,10-dihydrolysergic acid alkyl esters, and to provide improved ergot alkaloid drugs and intermediate products therefor.

### Summary of the Invention

According to one aspect, the present invention provides a process for producing *N*-6 demethylated 9,10-dihydrolysergic acid alkyl ester, comprising a step of *N*-6 demethylation of a 9,10-dihydrolysergic acid alkyl ester with chloroformate, characterized that the *N*-6 demethylation step is performed in the presence of an organic catalyst.

According to another aspect, the present invention provides a process for converting a compound of formula I (DHLSAlkyl) into a compound of formula II (DHLSAlkyl-F), 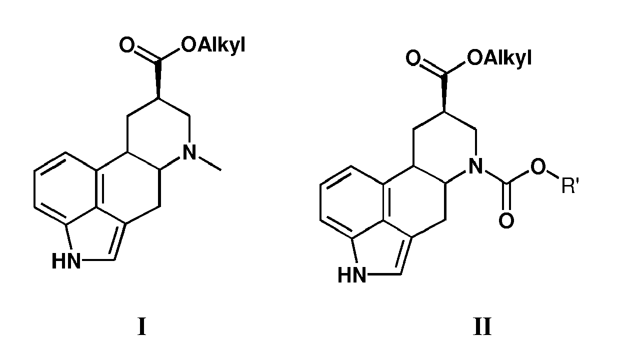 wherein R' is a substituted or unsubstituted hydrocarbon group, the process comprising the steps of:
■ providing and preferably maintaining a reaction medium free of water and/or HCl, and
■ reacting DHLSAlkyl with chloroformate being defined by the formula 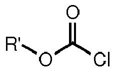 with R' having the aforementioned meaning in said reaction medium free of water and/or HCl, to obtain the compound of formula II (DHLSAlkyl-F).

In still another aspect, the present invention provides a process for producing N-6 demethylated 9,10-dihydrolysergic acid alkyl ester, comprising a step of *N*-6 demethylation of a 9,10-dihydrolysergic acid alkyl ester with chloroformate, wherein the ratio of chloroformate used in total compared to 9,10-dihydrolysergic acid alkyl ester used in total for the step of *N*-6 demethylation is less than 5 equivalents to 1 equivalent, respectively.

The process defined in the aspects above can be beneficially used in a process for the preparation of ergot alkaloid drugs.

Moreover, the present invention provides intermediate compounds of formula II (DHLSAlkyl-F), intermediate compounds of formula III (H-DHLSAlkyl), and ergot alkaloid drugs, respectively free of any one of impurities of formula 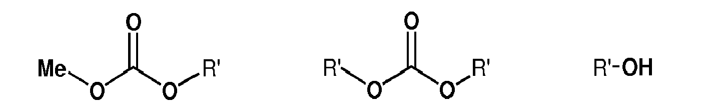 wherein R' is a substituted or unsubstituted hydrocarbon group.

The aforementioned compounds and drugs are obtained by providing corresponding reaction media respectively containing said compounds and drugs, e.g. the demethylation reaction medium from the *N*-6 demethylation of 9,10-dihydrolysergic acid alkyl ester of the invention, but being free, preferably entirely free of the afore-defined impurities. The desired compounds and drugs can then be isolated in pure form.

In still another aspect, the present invention encompasses the use of chloroformate defined by the formula 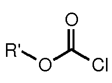 with R' having the aforementioned meaning for the *N*-6 demethylation of 9,10-dihydrolysergic acid alkyl esters in the manufacture of an intermediate product defined by formula II (DHLSAlkyl-F), an intermediate product defined by formula III (H-DHLSAlkyl), or ergot alkaloid drugs such as cabegoline and pergolide free of the aforementioned impurities.

The term "alkyl" used herein, if not specified otherwise, corresponds to the typical meaning of, for example, straight chain or branched chain, unsubstituted or substituted organic alkyl groups, possibly containing C=C double bonds and/or hetero atoms such as O, S, N with optional further substituents, suitably e.g. C1 to C6 alkyl, preferably methyl, ethyl, propyl, isopropyl, butyl, pentyl, hexyl, or the like.

The term "N-6 demethylation" used herein means replacement of methyl group by another group, more particularly it means replacement by a formate group being defined by 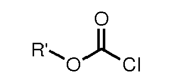 with R' having the aforementioned meaning, or a replacement by hydrogen.

Thus, the above-mentioned object is basically solved by the subject-matter of present claims 1, 5, 10, 12, 13 and 14. Preferred embodiments are set forth in sub-claims thereto.

### Brief Description of the Drawings

Fig. 1 shows process chemistry based on prior art, from which adaptations and optimizations according to an embodiment of the present invention become apparent;
Fig. 2 shows organocatalytic process chemistry replacing the inorganic bicarbonate system of Fig. 1, forming the basis for another embodiment of the present invention;
Fig. 3 illustratively shows a possible synthesis scheme for obtaining ergot alkaloid drugs, such as for example cabegoline or pergolide.
Fig. 4 shows a comparison of kinetics of DHLSMe conversion to DHLSMe-F for adapted and optimized standard, and for organocatalytic processes according to various embodiments of the present invention.

### Detailed Description of the Invention, its Advantages and Preferred Embodiments

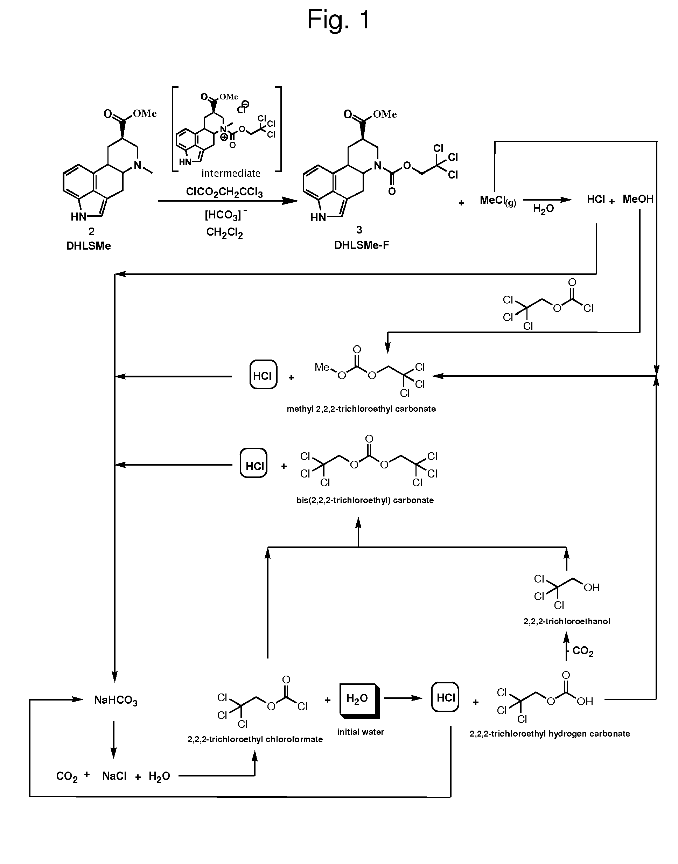

In the present invention, it was found that the synthesis system described by Crider et al. is not industrially applicable due to its unscalability from the laboratory scale to industrial scale. It was recognized that inorganic bicarbonates, as required in the synthesis route disclosed by Crider et al., exert a deleterious effect by entering a self-sustaining (driving, preserving) cycle of chemical reactions that produce and accumulate water, HCl and other side products such as organic carbonates in the system, which eventually blocks the demethylation reaction. Chemical reactions being presumably relevant in the reaction system of the demethylation step are shown in Fig. 1, as illustrated for the example where the chloroformate is embodied by trichloroethyl chloroformate (TCECF). Without proper adaptations and control of the reaction system, it occurs that per one cycle at least 3 molecules of water are released from an initial molecule of water (originating from the solvent or moisture) via release of 3 molecules HCl per cycle and its reaction with HCO₃⁻. Therefore, presence of water would be amplified and accumulated from initial water in the system during the reaction and would eventually block the demethylation reaction progress through released and co-accumulated HCl, the latter protonating the 6-N nitrogen atom of the basic ergoline structure and thus blocks it to react with the chloroformate (here TCECF). The water and HCl accumulation problems identified by the present inventors have found to become pronounced especially when the reaction system shall be scaled up.

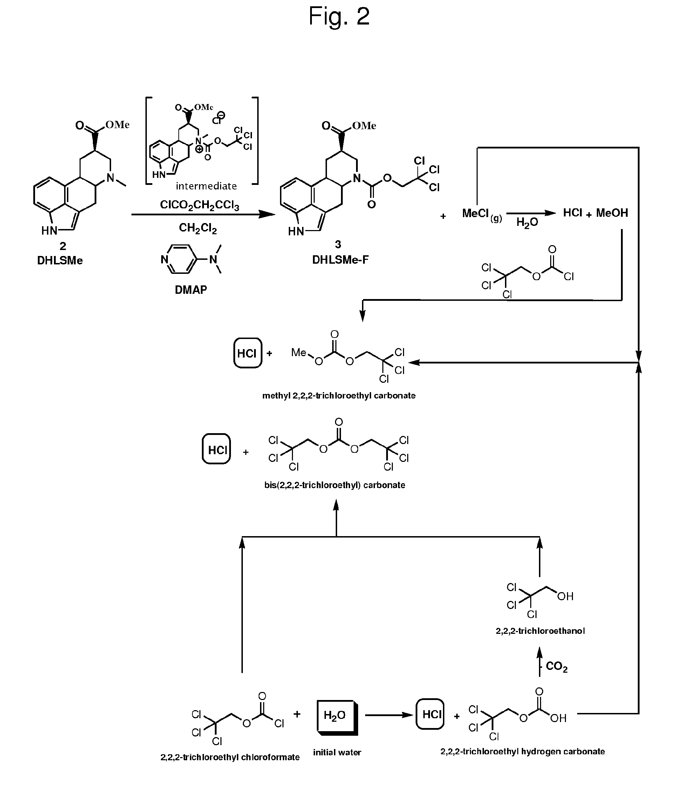

It was now surprisingly found, however, that when applying organic catalyst in the N-6 demethylation step, as shown in Fig. 2 by illustratively referring again to the use of trichloroethyl chloroformate (TCECF) reagent, a water release and thus water accumulation is prevented during its function. Consequently, HCl release and accumulation can be prevented or minimized as well. This leads to a significant increase of the yield of the product, especially on industrial scale, compared to the original procedure disclosed by Crider et al. Thus, according to one solution approach of the present invention, the N-6 demethylation step is performed in the presence of an organic catalyst. In this approach, potassium bicarbonate or other bicarbonates or possibly also other inorganic bases, and the problems associated therewith, can be surely avoided.

Moreover, the findings of the present invention provided another alternative solution approach of the problems associated with the synthesis system by Crider et al. Namely, if particular care is observed to ensure an absence and preferably an entire absence of initial water, the probability of HCl release and water accumulation reactions as shown in Fig. 1 is significantly reduced and minimized, even if an organic catalyst is not used. Therefore, providing a reaction medium free of water and/or HCl already from the beginning before the demethylation reaction is started to proceed by adding a relevant co-educt, does have a remarkable and - especially when viewed from a laboratory scale - unpredictable effect of significantly increasing the yield of the product. This effect becomes substantial particularly on an industrial scale, when inorganic bases such as bicarbonates are used as released acid scavengers.

The solution approaches of the present invention described above can be advantageously combined.

When an organic catalyst is used for the N-6 demethylation step in the process according to the present invention, typically a organic compound having a proton acceptor function is selected. Preferred organic catalyst according to the present invention is selected from the group consisting of sterically hindered organic amines, secondary and tertiary organic amines, aromatic amines and heterocyclic organic amines. Suitable examples of such organic amines include, but are not limited to triethylamine, pyridine and pyridine derivatives, substituted or unsubstituted forms of dialkylaminopyridine and particularly dimethylaminopyridine, N-ethylmorpholine, imidazole, N-ethyldiisopropylamine, N-ethylmorpholine triethylamine, 1,5-diazabicyclo[4.3.0]non-5-ene, and the like. The organic catalyst used according to the present invention is particularly selected form the group of compounds consisting of dimethylaminopyridine (DMAP) and DMAP analogues of the following general formulae: 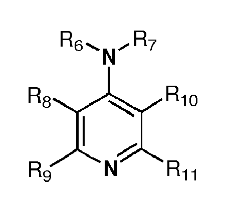 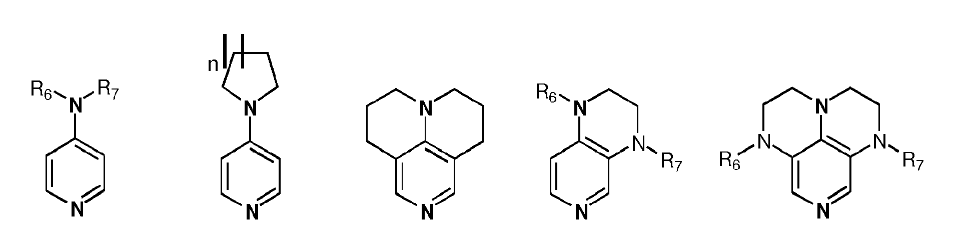 wherein R6 to R11 independently from each other denote H or C1 to C6 alkyl group, and n is 1, 2, 3, 4, 5 or 6.

Most preferably 4-(dimethylamino)pyridine (DMAP) 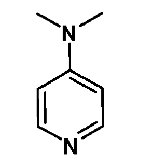 is used as the organic catalyst.

The use of DMAP and other analogs thereof as catalyst in organic synthesis such acylation reactions are described, for example, in D.J. Berry et al. ARKIVOC 2001 (i), pp. 201-226, and R. Murugan and E.F.V Scriven, Aldrichimica Acta 2003, 36, pp. 21 - 27.

When, in the alternative embodiment of the present invention, an organic catalyst is not used for the N-6 demethylation step or if it is only optional, it is important that a reaction medium free of water and/or HCl, more preferably entirely free of water is provided in advance of starting, or at the latest simultaneously with performing the actual demethylation step and specifically when converting a compound of formula I (DHLSAlkyl) into a compound of formula II (DHLSAlkyl-F). The importance of this condition is more pronounced, as the reaction system is scaled up from laboratory scale to bench scale and especially to industrial scale. This particularly applies when using a large scale at an amount of 9,10-dihydrolysergic acid alkyl ester (compound of formula I; DHLSAlkyl) above 1 kg, because scaling up increases the risk that any minute amounts of initial water is present in the system, which eventually accumulates further water molecules and releases further molecules of HCl per reaction cycle when bases like alkali metal bicarbonates, other than organic catalysts, are used. Thus, it has been found that if water present in the reaction system would be removed completely, release of HCl can be effectively prevented and thus problems of scaling up of the reaction system can be avoided even without using organic catalysts for the N-6 demethylation step. As particularly effective means to provide a reaction medium free of water and/or HCl, the medium can be subjected to purging with nitrogen or any other inert gas, and/or subjected to evacuation. This measure can be preferably carried out in advance of adding DHLSAlkyl into the reaction system,

Preferably, it is observed that a reaction medium free of water and/or HCl is not only provided in the starting materials or simultaneous with adding co-reactants, but also is maintained throughout the N-6 demethlyation reaction. For this purpose, it is preferred that a water and/or HCl removal step, such as degasing by nitrogen flow or purge or by vacuum evacuation, may be repeatedly performed. At least one water and/or HCl removal step is preferably performed after chloroformate has been added to a water-free solvent, by which measure a majority or all of possibly generated HCl is removed from the reaction system. Any remaining traces of HCl may be further captured by the base catalyst. Another suitable point of time for repeating a water and/or HCl removal step is when DHLSAlkyl is added and/or subsequent of its addition. Thus, according to preferred embodiment, the process of converting a compound of formula I into a compound of formula II comprises the steps of:
- providing a reaction medium free of water and/or HCl,
- before DHLSAlkyl is added, chloroformate is added to the reaction medium, and the reaction medium is refluxed,
- after chloroformate addition and medium reflux, the reaction medium is optionally but preferably purged with nitrogen, or is subjected to evacuation,
- DHLSAlkyl is then added to the obtained mixture, and
- the obtained mixture is subjected to reaction for time sufficient to give DHLSAlkyl-F (N-6 demethylated) product.

A preferred chloroformate reactant is defined by 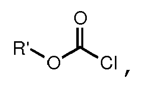 wherein R' is a substituted or unsubstituted hydrocarbon group, to give the corresponding demethylated DHLSAlkyl-F. Preferred R' residues are "alkyl", more preferably halogenated alkyl or vinyl. Particularly R' is defined by formula IV or V 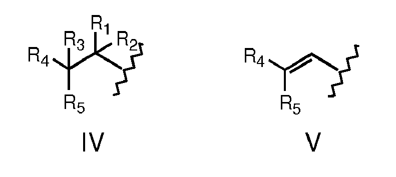 wherein R₁ and R₂, are the same or different and can be hydrogen atoms or are selected from halogens atoms (F, Cl, Br, I),
and wherein R₃, R₄, and R₅, are the same or different and can be hydrogen atoms or are selected from halogens atoms (F, Cl, Br, I), optionally substituted C₁ - C₈ alkyl, optionally substituted C₃-C₆ cycloalkyl, optionally substituted C₁ - C₈ alkenyl, and optionally substituted C₅-C₆ cycloalkenyl or aryl.

According to a particularly preferred embodiment of the present invention, both aspects of the invention are combined to provide an optimized reaction system which is most suitable for an up scaled N-6 demethylation of 9,10-dihydrolysergic acid alkyl esters. That is, a reaction medium free of water and/or HCl is provided, and the reaction medium is supplemented with an organic catalyst as described above at a desired point of time. A reaction medium containing chloroformate is preferably subjected to heat treatment, preferably under reflux, for some periods of time, for example at least 1, preferably at least 2 and more preferably at least 3 hours. Optionally, after cooling, the reaction medium can be purged with inert gas flow such as nitrogen purging and/or can be subjected to evacuation. Subsequently, organic catalyst as described above may be added at this point and may be mixed with the reaction medium for a period of time, optionally followed by another water and/or HCl removal step such as inert gas purging and/or evacuation. In accordance with the preferred embodiment, not before then the deduct DHLSAlkyl is added to start with the actual demethylation reaction.

Subsequent to the N-6 demethylation reaction, the carbamate group of the compound of formula II (DHLSAlkyl-F) can be subjected to further reactions in accordance with the options and choice of the person skilled in the art, such as the intended further synthesis route to obtain a desired ergot alkaloid drug. The carbamate group of the compound of formula II (DHLSAlkyl-F) may be cleaved by methods known in the art, to give the compound of formula III (H-DHLSAlkyl). The compound of formula III (H-DHLSAlkyl) can likewise be subjected to further reaction and synthesis steps to obtain various ergot alkaloid drugs, by methods known or apparent to the person skilled in the art.

A particularly advantageous embodiment of the present invention resides in that the molar ratio of co-reactant chloroformate used in total compared to 9,10-dihydrolysergic acid alkyl ester used in total for the step of N-6 demethylation can be significantly reduced in comparison with the prior art. Surprisingly, the process of present invention enables a reduction of the aforementioned ratio to less than 5 equivalents chloroformate to 1 equivalent of 9,10-dihydrolysergic acid alkyl ester in the N-6 demethylation step.

Hence, the present invention provides a robust, reliable, economic and scalable process for N-6 demethylation of 9,10- dihydrolysergic acid alkyl esters with chloroformate, with particular significance of increased yield of the product on an industrial scale. Thus, the process according to the present invention is particularly suitable to be performed at a large scale by using an amount of 9,10- dihydrolysergic acid alkyl ester above 1 kg, more preferably above 2 kg and even above 4 kg. Following the concept of the present invention enables the demethylation step for obtaining an intermediate compound of formula II (DHLSAlkyl-F) to be terminated in less than 24 hours.

Therefore, further particularly advantageous aspect of the present invention is the use of the process according to the present invention as described above in a process for the preparation of ergot alkaloid drugs. The advantages of the present invention are variable for the whole preparation scheme of total ergot alkaloid drug synthesis.

A further advantage and particularly preferred embodiment of the present invention is that the intermediate compounds of DHLSAlkyl-F or H-DHLSAlkyl, and accordingly any final ergot alkaloid drug obtained therefrom, are feasible which are free, beneficially entirely free of impurities of the following formulae, which otherwise would appear by following conventional synthesis routes: 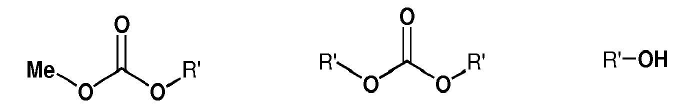 wherein R' is the hydrocarbon group derived from the chloroformate reactant. Specifically, R' is as defined above.

Based on the preferred embodiment of the present invention to use 2,2,2-trichloroethyl chloroformate (TCECF) as chloroformate for the N-6 demethylation of 9,10-dihydrolysergic acid alkyl esters, the intermediate product defined by either formula II or III (DHLSAlkyl-F or H-DHLSAlkyl), and the ergot alkaloid drugs are free and preferably entirely free of the following corresponding chlorinated impurities:

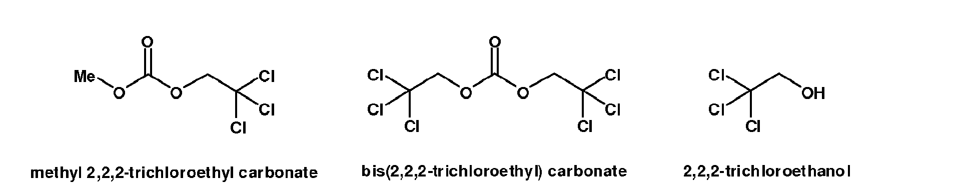

As used herein, "entirely free" means non-detectable by methods like GC, HPLC or mass spectrometry. As a suitable reference method for detectability, GC can be used.

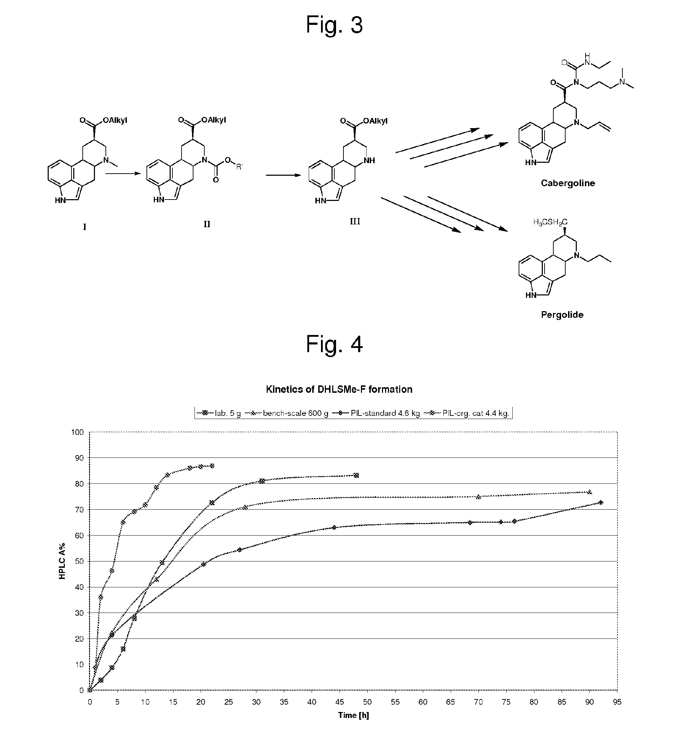

A possible synthesis scheme for obtaining ergot alkaloid drugs, such as preferably cabegoline or pergolide, are illustratively shown in Fig. 3, wherein the synthesis routes proceed by using compounds of formulae I, II, and III. According to the preferred embodiment, alkyl is methyl (Me), and thus the synthesis of ergot alkaloid drugs proceeds via DHLSMe-F and H-DHLSMe.

The present invention will be described in more detail in the following by reference to illustrative examples, which however are given for exemplary purposes only and shall not be interpreted in any limiting manner. Rather, the skilled person may use modifications or find equivalents within ordinary skill. For example, the use of the preferred chloroformate 2,2,2-trichloroethyl chloroformate (TCECF) is described in the examples and the Figures, while the skilled person will appreciate that alternative chloroformates as generically described above can be correspondingly used as well.

### Reference Examples

### Repeated literature procedure from A. M. Crider et al., J. Pharm. Sci. 1981, 70, 1319-1321.

### 6-Nor-6-(2,2,2-trichloroethoxycarbonyl)-9,10-dihydrolysergic Acid Methyl Ester

A mixture of 9,10-dihydrolysergic acid methyl ester (0.425g, 0.0015 mole), potassium bicarbonate (0.729 g, 0.0073 mole), and 2,2,2,trichloroethyl chloroformate (0.619 g, 0.0029 mole) in 60 mL of absolute methylene chloride was refluxsed for 24 hr. 2,2,2-Trichloroethyl chloroformate (1.48 g, 0.0070 mole) was added and refluxing was continued for an additional 24 hr. The reaction mixture was cooled, filtered, and evaporated under reduced pressure to yield a white solid. Recrystalization from absolute methanol gave 0.602 g (90 %) of crystaline product, mp 208-210°; IR(KBr): 3485 (N-H, indole) and 1740 (C=O, ester an carbamate) cm ⁻¹; NMR (dimethyl sulfoxide-d₆): δ 3.70 (s, 3H, COOCH₃), 4.90 (s, 2H; OCH₂CCl₃), 6.67-7.20 (m, 4H, aromatic), and 7.27 (s, 1H, NH). Anal.-Calc. For C₁₉H₁₉Cl₃N₂O₄: C, 51.19; H, 4.30; Cl, 23.86; N, 6.29. Found: C, 51.14; H, 4.20; Cl, 24.21; N, 6.13.

Two batches were performed on 2.13 g scale:
2.13 g DHLSMe
3.645 g KHCO₃
300 mL DCM
2.01 mL TKEKF reflux 24 h
4.80 mL TKEKF reflux 24 h

### First batch

### Kinetics of the reaction:

| **t (h)** | **DHLSMe** [HPLC A%] | **DHLSMeF** [HPLC A%] |
|---|---|---|
| 4 | 93.69 | 5.55 |
| 24 | 81.70 | 15.54 |
| 29 | 73.71 | 25.32 |
| 48 | 62.95 | 35.85 |

### Second batch

### Kinetics of the reaction:

| **t (h)** | **DHLSMe** [HPLC A%] | **DHLSMeF** [HPLC A%] |
|---|---|---|
| 4 | 93.08 | 5.53 |
| 24 | 83.77 | 15.53 |
| 29 | 73.53 | 25.54 |
| 48 | 49.38 | 50.62 |

The reaction mixture was cooled, filtered, and evaporated under reduced pressure to yield a white solid.

### Recrystalization from absolute methanol gave:

First batch: 1.02 g (30 %) HPLC: 99.67 area%
Second batch: 1.16 g (35 %) HPLC: 99.40 area%
of crystalline product.

### Example 1

### Optimized chemical reaction system, lab scale (5.0 g)

5.0 g (17.58 mmole) of 9,10-dihydrolysergic acid methyl ester was dissolved in 200 mL of absolute methylene chloride, after 7.2 g (85.8 mmol) of sodium bicarbonate were added and mixture was heated to reflux. Then 16.5 mL (25.39 g, 120 mmol) of 2,2,2,trichloroethyl chloroformate were slowly added in 24 hours. After complete addition the reaction mixture was refluxed further for 24 hours. After 48 hours the reaction mixture was cooled, filtered over aluminium oxide (10 g), washed with 150 mL of methylene chloride and evaporated under reduced pressure and 50 mL of absolute methanol were added and stirred for 2 hours. Crystalline product was filtered and dried to yield a 5.92 g (76 %) of product as a white solid.

### Kinetics of the reaction:

| t [h] | DHLSMe [HPLC A%] | DHLSMe-F [HPLC A%] |
|---|---|---|
| 2 | 95.35 | 3.95 |
| 4 | 90.45 | 8.68 |
| 6 | 84.00 | 16.00 |
| 8 | 71.59 | 27.81 |
| 13 | 49.95 | 49.41 |
| 22 | 23.98 | 72.58 |
| 31 | 17.27 | 81.09 |
| 48 | 15.69 | 83.25 |

### Example 2

### Optimized chemical reaction system, bench-scale (600 g):

600 g (2.11 mole) of 9,10-dihydrolysergic acid methyl ester was dissolved in 24 L of absolute methylene chloride, after 864 g (10.3 mmol) of sodium bicarbonate were added. Then 2.515 L (3.87 kg, 18.3 mol) of 2,2,2,trichloroethyl chloroformate were added in four portions (t₀ₕ = 640 mL, t_{11.5h} = 625 mL, t₂₈ₕ = 625 mL, t₇₀ₕ = 625 mL). Reaction mixture was refluxed for 90 hours, then the reaction mixture was cooled, filtered over aluminium oxide (6.2 kg), washed with 20 L of methylene chloride and evaporated under reduced pressure to 4 L. After 4.5 L of n-hexane were added and stirred for 2 hours at 0 °C. Crystalline product was filtered and dried to yield a 553 g (59 %) of white solid.

### Kinetics of the reaction:

| t [h] | DHLSMe [HPLC A%] | DHLSMe-F [HPLC A%] |
|---|---|---|
| 4 | 75.15 | 22.47 |
| 12 | 54.02 | 42.98 |
| 28 | 26.53 | 71.05 |
| 70 | 21.45 | 75.04 |
| 72.5 | 19.45 | 71.35 |
| 90 | 17.90 | 76.80 |

### Example 3

### Optimized chemical reaction system, industrial scale (4.6 kg):

A dry nitrogen inertized 250 L reactor was charged with 4.6 kg DHLSMe, 6.6 kg sodium bicarbonate and 210 kg of dry dichloromethane. Then 2,2,2-trichloroethyl chloroformate (TCECF) (7.0 kg) was charged into the reactor. The mixture was heated to reflux and stirred at reflux for 92 hours. Additional quantity (18.5 kg) of 2,2,2-trichloroethyl chloroformate (TCECF) was charged into the reactor during the stirring at reflux. After the reaction mixture was cooled to 10 ± 5 °C and filtered through a pad of alumina. Fractions containing a product were collected, concentrated and cooled 10 ± 5 °C. Then heptane is added to the concentrated solution and the precipitated product was collected by centrifugation. After drying 4.1 kg (56 %) of DHLSMe-F is obtained.

### Kinetics of the reaction:

| t [h] | DHLSMe [HPLC A%] | DHLSMe-F [HPLC A%] |
|---|---|---|
| 0 | 100 | 0 |
| 4 | 78,16 | 21,27 |
| 20,5 | 50,98 | 48,74 |
| 27 | 45,35 | 54,34 |
| 44 | 36,73 | 63,01 |
| 68,5 | 34,73 | 64,98 |
| 74 | 34,53 | 65,18 |
| 76,5 | 34,2 | 65,43 |
| 92 | 26,84 | 72,7 |

### Example 4

### Organocatalytic chemical reaction system, industrial scale (4.4 kg):

The following reaction scheme shows the principle applied in Example 4 of *N*-6 demethylation of 9,10-dihydrolysergic acid alkyl esters with chloroformates in the presence of DMAP.

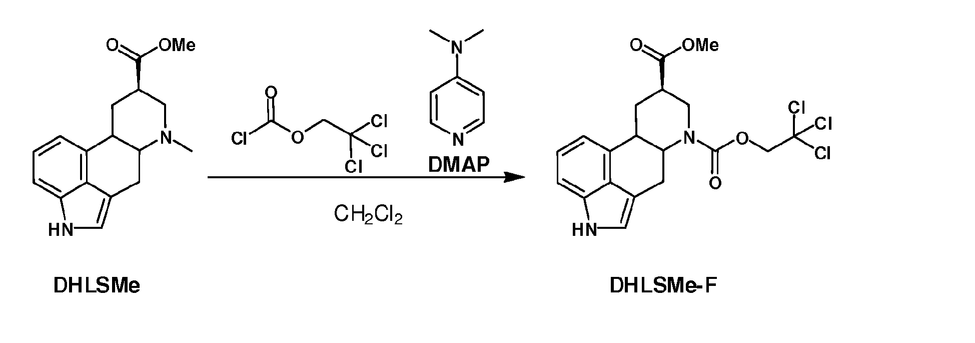

In the present example, 2,2,2-trichloroethyl chloroformate was used (i.e. R' is 2,2,2-trichloroethyl).

A 250 L reactor containing a 186 kg of dry dichlorometane was charged with 15.4 kg 2,2,2-trichloroethyl chloroformate (TCECF) at 20 ± 5 °C. The mixture was heated to reflux and refluxed for at least 3 hours. After the mixture was cooled to 20 ± 5 °C. The ractor was purged with nitrogen for at least 15 min. and charged with 88 g of DMAP at 20 ± 5 °C. The obtained mixture was then stirred at 20 ± 5 °C for at least 1 hour. The ractor was purged with nitrogen for at least 15 min. and charged with 4.4 kg of DHLSMe at 20 ± 5 °C. The obtained mixture was then heated to reflux and refluxed for 22 hours. After the reaction mixture was cooled to 10 ± 5 °C and filtered through a pad of alumina. Fractions containing a product were collected, concentrated and cooled 10 ± 5 °C. Then heptane is added to the concentrated solution and the precipitated product was collected by centrifugation. After drying 5.25 kg (76 %) of DHLSMe-F is obtained. DHLSMe-F assay is 100 % determined by HPLC.

### Kinetics of the reaction:

| t [h] | DHLSMe [HPLC A%] | DHLSMe-F [HPLC A%] |
|---|---|---|
| 0 | 100 | 0 |
| 1 | 90,87 | 8,71 |
| 2 | 63,55 | 36,10 |
| 4 | 53,1 | 46,32 |
| 6 | 34,28 | 65,07 |
| 8 | 30,2 | 69,14 |
| 10 | 27,12 | 71,77 |
| 12 | 20,38 | 78,44 |
| 14 | 15,98 | 83,34 |
| 18 | 12,39 | 86,01 |
| 20 | 12,08 | 86,62 |
| 22 | 11,67 | 86,90 |

### Example 5

Molar ratios of co-reactants, kinetics any yields of the chemical reaction systems used in the Reference Example and in the Examples 1 to 4 were followed and monitored.

Advantages of organocatalytic process over standard process are presented in Table 1 which presents comparison of yields, reaction time and reagent stoichiometry (molar ratio between TCECF and DHLSMe).

**Table 1**

| | **Type and scale of reaction** | **Reaction time [h]** | **Yield [%]** | **Molar ratio TCECF/DHLSMe** |
|---|---|---|---|---|
| Reference Ex. | Literature data, lab scale (0.425 g) | 48 | 90 | 6.6 : 1 |
| Reference Ex. | Repeated literature data, lab scale (2.13 g) | 48 | 30-35 | 6.6 : 1 |
| Example 1 | Optimized, lab scale (5.0 g) | 48 | 76 | 6.8 : 1 |
| Example 2 | Optimized, bench-scale (600 g) | 90 | 59 | 8.7 : 1 |
| Example 3 | Optimized, industrial scale (4.6 kg) | 92 | 57 | 7.4 : 1 |
| Example 4 | Organocatalysis, industrial scale (4.4 kg) | **22** | **76** | **4.7 : 1** |

Further, comparison of kinetics for conversion of DHLSMe to DHLSMe-F is presented in Figure 4 (scale is based on the quantity of starting 9,10-dihydrolysergic acid methyl ester = DHLSMe entering the reaction) for adapted and optimized versions of standard reaction using inorganic bicarbonate base on lab-scale (5 g), bench-scale (600 g), industrial-scale = PIL-standard (4.6 kg) and by applying organocatalysis with DMAP on industrial scale = PIL-org. cat. (4.4 kg).

From Figure 4 significantly improved effects of the process according to the present invention, especially when using organocatalysis over standard process are clearly visible. Example 4 provides highest conversion of DHLSMe to DHLSMe-F in shortest time.

## Claims

1. A process for producing *N*-6 demethylated 9,10-dihydrolysergic acid alkyl ester, comprising a step of *N*-6 demethylation of a 9,10-dihydrolysergic acid alkyl ester with a chloroformate, wherein the *N*-6 demethylation step is performed in the presence of an organic catalyst.

2. The process according to claim 1, **characterized in that** the chloroformate is defined by the formula 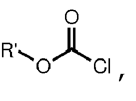 wherein R' is a substituted or unsubstituted hydrocarbon group, particularly wherein R' is defined by formula IV or V 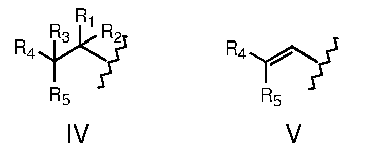 wherein R₁ and R₂, are the same or different and can be hydrogen atoms or are selected from halogens atoms (F, Cl, Br, I),
and wherein R₃, R₄, and R₅, are the same or different and can be hydrogen atoms or are selected from halogens atoms (F, Cl, Br, I), optionally substituted C₁ - C₈ alkyl, optionally substituted C₃-C₆ cycloalkyl, optionally substituted C₁ - C₈ alkenyl, and optionally substituted C₅-C₆ cycloalkenyl or aryl.

3. The process according to any one of the preceding claims, **characterized in that** the organic catalyst is an organic amine, preferably selected from the group consisting of sterically hindered organic amines, secondary and tertiary organic amines, and heterocyclic organic amines.

4. The process according to any one of the preceding claims, **characterized in that** the organic catalyst is selected from the group of compounds consisting of dimethylaminopyridine (DMAP) and DMAP analogues of the following general formulae: 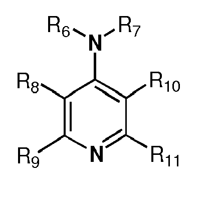 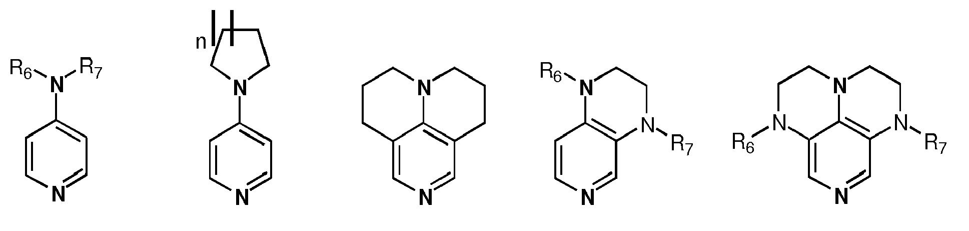 wherein R6 to R11 independently from each other denote H or C1 to C6 alkyl group, and n is 1, 2, 3, 4, 5 or 6,
wherein preferably the organic catalyst is 4-(dimethylamino)pyridine (DMAP).

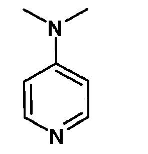

5. A process for converting a compound of formula I (DHLSAlkyl) into a compound of formula II (DHLSAlkyl-F), 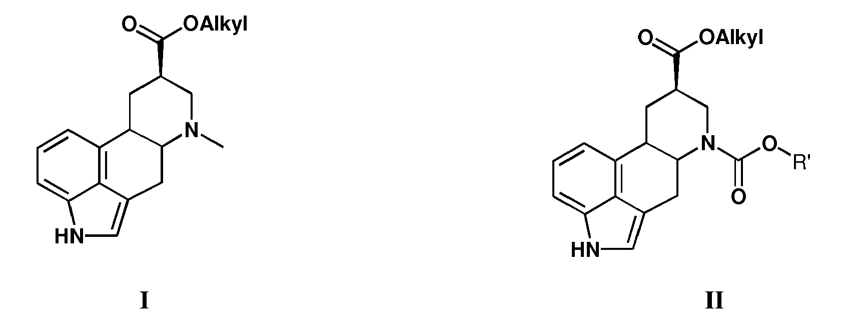 wherein R' is a substituted or unsubstituted hydrocarbon group, preferably a group as defined in claim 2 by formula IV or V,
the process comprising the steps of:
■ providing a reaction medium free of water and/or HCl, and
■ reacting DHLSAlkyl with chloroformate being defined by the formula 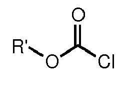 in said reaction medium free of water and/or HCl, to obtain the compound of formula II (DHLSAlkyl-F).

6. The process according to claim 5, wherein at least one additional water and/or HCl removal step is performed.

7. The process according to claim 5 or 6, wherein
a) before DHLSAlkyl is added, said chloroformate is added to the reaction medium, and the reaction medium is refluxed,
b) the reaction medium is optionally purged with nitrogen or subjected to evacuation,
c) DHLSAlkyl is added to the above mixture resulting from a) and optionally b)
d) reacting the obtained mixture for a time sufficient to give *N*-6 demethylated product.

8. The process according to any one of claims 5 to 7, wherein after said chloroformate is added to the reaction medium and preferably the reaction medium is optionally purged with nitrogen or subjected to evacuation, an organic catalyst, which is preferably defined as in claim 3 or 4, is added to the mixture, and optionally the mixture is subsequently purged with nitrogen or subjected to evacuation, before DHLSAlkyl is added.

9. The process according to any one of claims 5 to 8, further comprising the step of cleaving of the carbamate group of the compound of formula II (DHLSAlkyl-F) to give the compound of formula III (H-DHLSAlkyl):

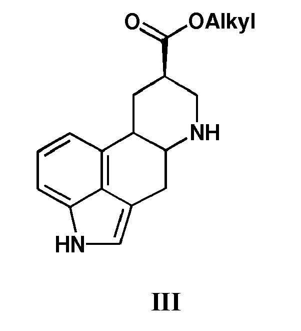

10. A process for producing *N*-6 demethylated 9,10-dihydrolysergic acid alkyl ester, comprising a step of *N*-6 demethylation of a 9,10-dihydrolysergic acid alkyl ester with a chloroformate, **characterized** that the ratio of chloroformate used in total compared to 9,10-dihydrolysergic acid alkyl ester used in total for the step of *N*-6 demethylation is less than 5 equivalents to 1 equivalent, respectively.

11. The process according to any one of the preceding claims, which is performed at a large scale by using an amount of 9,10-dihydrolysergic acid alkyl ester (compound of formula I; DHLSAlkyl) above 1 kg, preferably wherein the demethylation step to obtain the compound of formula II (DHLSAlkyl-F) is terminated in less than 24 hours.

12. A process for the preparation of ergot alkaloid drugs, comprising a process as defined in any one of claims 1 to 11.

13. Intermediate compound of formula II (DHLSAlkyl-F), intermediate compound of formula III (H-DHLSAlkyl), or ergot alkaloid drug, 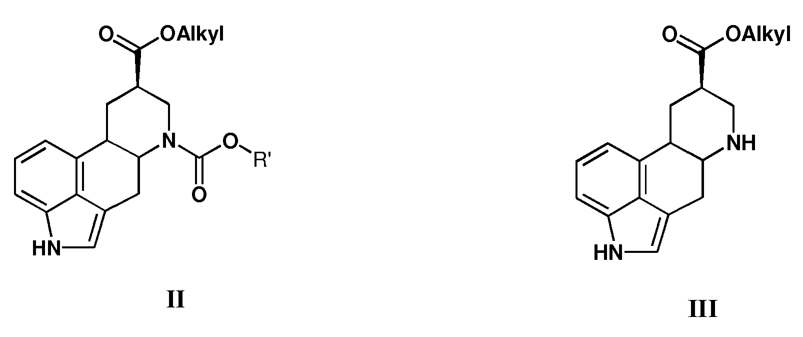 said intermediate product defined by formula II (DHLSAlkyl-F), said intermediate product defined by formula III (H-DHLSAlkyl), or said ergot alkaloid drugs respectively being free of any one of impurities of formula 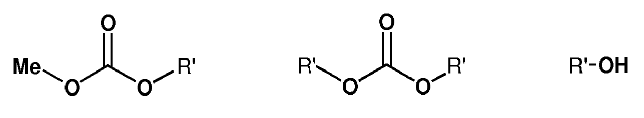 wherein R' is a substituted or unsubstituted hydrocarbon group, particularly wherein R' is defined by formula IV or V 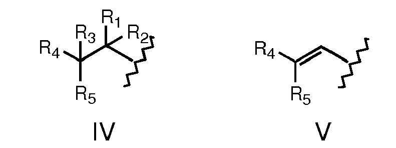 wherein R₁ and R₂, are the same or different and can be hydrogen atoms or are selected from halogens atoms (F, Cl, Br, I),
and wherein R₃, R₄, and R₅, are the same or different and can be hydrogen atoms or are selected from halogens atoms (F, Cl, Br, I), optionally substituted C₁ - C₈ alkyl, optionally substituted C₃-C₆ cycloalkyl, optionally substituted C₁ - C₈ alkenyl, and optionally substituted C₅-C₆ cycloalkenyl or aryl.

14. A use of a chloroformate defined by the formula 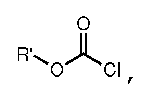 wherein R' is a substituted or unsubstituted hydrocarbon group, for the *N*-6 demethylation of 9,10-dihydrolysergic acid alkyl esters in the manufacture of an intermediate product defined by formula II (DHLSAlkyl-F), an intermediate product defined by formula III (H-DHLSAlkyl), or ergot alkaloid drugs, 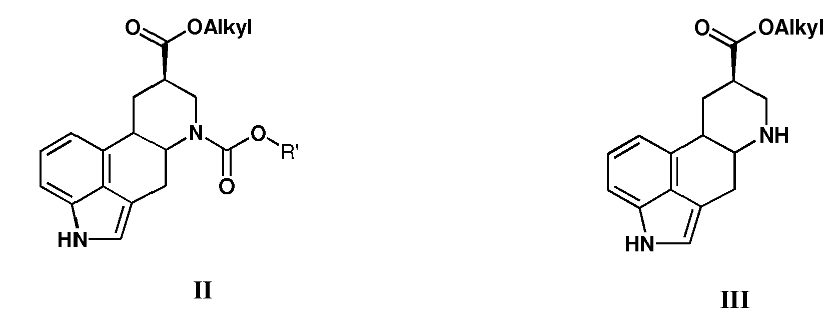 said intermediate product defined by formula II (DHLSAlkyl-F), said intermediate product defined by formula III (H-DHLSAlkyl), or said ergot alkaloid drugs respective being free of impurities of formula 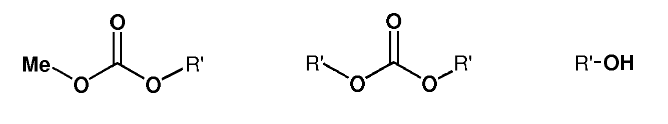 wherein R' has the same meaning as in the formula of the chloroformate used.

15. The process according to claim 12, the ergot alkaloid drug according to claim 13 or the use according to claim 14, wherein the ergot alkaloid drug is cabegoline or pergolide.
